Europäisches Patentamt

European Patent Office

Office européen des brevets

(11) Veröffentlichungsnummer: **0 158 221**
**B1**

## (12) EUROPÄISCHE PATENTSCHRIFT

(45) Veröffentlichungstag der Patentschrift:
**10.01.90**

(21) Anmeldenummer: **85103628.5**

(22) Anmeldetag: **27.03.85**

(51) Int. Cl.⁴: **C 07 C 31/30,** C 07 C 29/80, C 07 C 29/70

(54) Verfahren zur Herstellung von wasserfreiem Kalium-tert.-butoxid.

(30) Priorität: **07.04.84 DE 3413212**

(43) Veröffentlichungstag der Anmeldung:
**16.10.85 Patentblatt 85/42**

(45) Bekanntmachung des Hinweises auf die Patenterteilung:
**10.01.90 Patentblatt 90/2**

(84) Benannte Vertragsstaaten:
**AT BE CH DE FR GB IT LI LU NL SE**

(56) Entgegenhaltungen:
**EP-A- 0 091 425**
**GB-A- 2 088 734**
**US-A- 3 418 383**

(73) Patentinhaber: **HÜLS AKTIENGESELLSCHAFT,**
**Paul-Baumann-Strasse 1, D-4370 Marl 1 (DE)**

(72) Erfinder: **Matthes, Reinhard, Dr., Untere Dorfstrasse 71,**
**D-7888 Rheinfelden (DE)**
Erfinder: **Vahlensieck, Hans-Joachim, Dr., Im Habiken 2,**
**D-7867 Wehr (DE)**

## Beschreibung

Gegenstand der vorliegenden Erfindung ist ein Verfahren zur Herstellung von wasserfreiem Kalium-tert.-butoxid durch Umsetzung von wässriger Kalilauge mit tert.-Butylalkohol in einer mit Füllkörpern gefüllten Destillationskolonne, wobei das Wasser unter Mitverwendung eines Schleppmittels über Kopf abdestilliert wird und im Sumpf der Kolonne, der auf Siedetemperatur gehalten wird, eine alkoholische Lösung des Kalium-tert.-butoxids anfällt, die kontinuierlich abgezogen wird und anschließend zur Gewinnung des Kaliumsalzes aufgearbeitet wird.

Die Herstellung von Kalium-tert.-butoxid, das allgemein als Kalium-tert.-butylat bezeichnet wird, aus metallischem Kalium mit wasserfreiem tert.-Butylalkohol, wird in Houben-Weyl, Band IV/2 (1963), S. 7/8 beschrieben. Dabei wird auch auf die Möglichkeit des Arbeitens in einem inerten Lösungsmittel hingewiesen. Diese Arbeitsweise hat jedoch den Nachteil der schwierigen Handhabbarkeit des dabei eingesetzten metallischen Kaliums und der aufwendigen Herstellung des für eine einwandfreie Qualität des Endprodukts notwendigen absolut wasserfreien tert.-Butylalkohols.

Auch bei der bekannten Umalkoholisierung eines niederen Alkoholats mit dem gewünschten höheren Alkohol benötigt man im Falle der Herstellung von wasserfreiem Kalium-tert.-butoxid einen absolut wasserfreien tert.-Butylalkohol, dessen Reinherstellung sehr aufwendig ist. Zudem liegt bei der Umalkoholisierung niederer Kaliumalkoholate mit tert.-Butylalkohol das Gleichgewicht sehr stark auf der Seite der Ausgangsstoffe, so daß nur ein hoher Überschuß an tert.-Butylalkohol zu vernünftigen Ausbeuten führt. Dieser Überschuß bedingt ein Umkochen großer Butanolmengen wodurch sich der Energieaufwand dieser Verfahrensweise beträchtlich erhöht.

Die vorstehenden Nachteile werden durch die in der DE-PS 968 903 beschriebene Verfahrensweise weitgehend aus dem Wege geräumt. Dort wird das Reaktionswasser azeotrop abdestilliert und damit dem System entzogen. Dieses Verfahren hat jedoch den Nachteil, daß bei der Herstellung von Kalium-tert.-butoxid die sich auf den Kolonnen-Füllkörpern absetzenden Ablagerungen von fester KOH zur Verstopfung der Kolonne führen können. Eine Behebung dieses Nachteils ist zwar durch Einsatz von kleinen Mengen an Kalilauge möglich, jedoch wird dadurch die Ausbeute wesentlich verringert.

Die in der DE-PS 968 903 beschriebene Arbeitsweise erwähnt zwar das azeotrope Abdestillieren des Alkohols mit Hilfe eines Schleppmittels. Bei der dort geschilderten Arbeitsweise muß jedoch der Alkohol des abdestillierten Wasser/Alkohol-Azeotrops konstant erneuert werden, wozu eine aufwendige Rückgewinnung notwendig ist.

Es bestand deshalb die Aufgabe, die Herstellung von wasserfreiem Kalium-tert.-butoxid aus Kalilauge und tert.-Butylalkohol so zu führen, daß man die Umsetzung in einer Destillationskolonne kontinuierlich ohne Verstopfung oder Krustenbildung durchführen kann und die nicht umgesetzten Einsatzstoffe in einfacher Weise wieder zurückgewinnen kann. Weiterhin soll bei der neuen Arbeitsweise ein möglichst reines Endprodukt erhalten werden, dessen KOH-Gehalt möglichst gering, vorzugsweise unter 1 Gew.-% liegt.

In Erfüllung dieser Aufgabe wurd nun ein Verfahren zur Herstellung von wasserfreiem Kalium-tert.-butoxid durch Umsetzung von wässriger Kalilauge mit tert.-Butylalkohol in einer mit Füllkörpern gefüllten Destillationskolonne, Abdestillieren des Wassers unter Mitverwendung eines Schleppmittels und Abziehen der im Sumpf der Kolonne anfallenden alkoholischen Lösungen des Kalium-tert.-butoxids, wobei aus dieser abgezogenen Lösung anschließend in an sich bekannter Weise das wasserfreie Salz isoliert wird, gefunden, das durch die im Anspruch 1 genannten Maßnahmen a) bis c) gekennzeichnet ist.

Bei Durchführung der erfindungsgemäßen Verfahrensweise erhält man keine Verstopfungen in der Destillationskolonne und man kann den eingesetzten Alkohol fast vollständig ohne aufwendiges Aufarbeiten dem Prozeß wieder zurückführen. Es wird ein reines, wasserfreies Produkt erhalten, dessen KOH-Gehalt unter 1 Gew.-% liegt.

Die bei dem Verfahren eingesetzte wässrige Kalilauge ist zweckmäßigerweise eine konzentrierte Lauge, aus der bei den Reaktionstemperaturen festes KOH noch nicht ausfällt. Laugen mit KOH-Gehalten um 50 Gew.-% sind durchaus einsetzbar; der KOH-Gehalt kann zwar auch geringer sein, jedoch sind dann entsprechend größere Mengen an Wasser abzudestillieren. Aus diesem Grunde ist der Einsatz von Kalilaugen mit KOH-Gehalten unter 38 Gew.-% nicht empfehlenswert.

Der tert.-Butylalkohol braucht bei der erfindungsgemäßen Arbeitsweise nicht absolut wasserfrei zu sein. Wassergehalte unter 11,76% (Zusammensetzung des Azeotrops tert.-Butylalkohol/ Wasser) stören die erfindungsgemäße Verfahrensweise praktisch nicht. Bevorzugt soll der tert.-Butylalkohol jedoch einen Wassergehalt unter 0,1 Gew.-% besitzen.

Der tert.-Butylalkohol wird in einer solchen Menge eingesetzt, daß der gleichzeitig als Lösungsmittel für das erhaltene Kalium-tert.-butoxid dient. Die Menge soll dabei so groß gewählt werden, daß im Sumpf der Destillationskolonne eine 10 bis 18%ige, vorzugsweise eine 10 bis 15%ige Lösung des Kaliumsalzes vorliegt.

Die Menge des tert.-Butylalkohols soll weiterhin so gewählt werden, daß beim Sieden des Sumpfes der Anteil des tert.-Butylalkohols in dem in der Kolonnenmitte befindlichen Gasgemisch zwischen 50 und 90 Gew.-% liegt. Der restliche Anteil des dort befindlichen Gasgemischs besteht überwiegend aus Cyclohexan oder Hexan. Die Menge des Hexans bzw. Cyclohexans muß dementsprechend so gewählt werden, daß der genannte Anteil in dem Gasgemisch in der Kolonnenmitte vorliegt.

Der tert.-Butylalkohol wird im allgemeinen in flüssiger Form eingesetzt und auf den Kopf der Kolonne aufgegeben. Bei Anwendung der speziellen Arbeitsweise des Anspruchs 4 – d.h., wenn ein Teil des als Lösungsmittel eingesetzten tert.-Butylalkohols durch Cyclohexan oder Hexan ersetzt ist – sind die Anteile von Hexan oder Cyclohexan in dem Gasgemisch entsprechend höher und das Lösungsmittel für das Kalium-tert.-butoxid in dem Sumpf der Kolonne besteht teilweise ebenfalls aus Cyclohexan oder Hexan.

Die genannten Bedingungen für die einzusetzenden Mengen an tert.-Butylalkohol sind im allgemeinen dann erfüllt, wenn dieser in der 6 bis 20-fachen, vorzugsweise 9 bis 20-fachen Volumenmenge, bezogen auf die wässrige Kalilauge, eingesetzt wird.

Bei den erfindungsgemäßen Bedingungen der Zusammensetzung des Gasgemischs in der Kolonnenmitte und der Konzentration des siedenden Sumpfes an Kalium-tert.-butoxid ergibt sich am Kolonnenkopf ein Gasgemisch, das zu 20 bis 50 Gew.-% aus tert.-Butylalkohol und zu 50 bis 80 Gew.-% aus Cyclohexan oder Hexan, sowie aus Wasserdampf besteht. Je nach dem Wassergehalt des Gemischs beträgt die Kopftemperatur zwischen 65 und 75°C (bei Verwendung von Hexan anstelle von Cyclohexan 59 bis 69°C); der Wassergehalt des Gemischs liegt damit im allgemeinen unterhalb der Menge, die dem ternären Azeotrop Cyclohexan/tert.-Butylalkohol/Wasser entspricht, das einen Siedepunkt von 65°C besitzt (bei Verwendung von Hexan anstelle von Cyclohexan beträgt der Siedepunkt 59°C). Das in dem genannten Temperaturintervall über Kopf abgehende Gemisch – demzufolge kein reines Azeotrop – wird zweckmäßigerweise kondensiert, wobei es sich in eine wässrige und eine organische Phase auftrennt.

Die anfallende wässrige Phase enthält sowohl das in den Ausgangsprodukten enthaltene Wasser als auch das bei der Umsetzung entstehende Reaktionswasser. In dieser Phase kann tert.-Butylalkohol bis zu einem Gehalt an 10 Gew.-% gelöst enthalten sein.

Die über Kopf abgehende organische Phase wird zweckmäßigerweise nach ihrer Kondensation wieder der Destillationskolonne zurückgeführt, vorzugsweise auf den Kopf der Kolonne. Sie enthält überwiegend das Schleppmittel und den nicht im Wasser gelösten Anteil des überdestillierten tert.-Butylalkohols.

In der Kolonne wird das dort entstehende Reaktionsprodukt durch den überschüssigen tert.-Butylalkohol als Lösung in dem Gemisch tert.-Butylalkohol/Hexan bzw. Cyclohexan in den Sumpf der Kolonne gespült, der während der gesamten Umsetzung auf Siedetemperatur gehalten wird. In dem Sumpf liegt das Kalium-tert.-butoxid dann als 10 bis 18%ige Lösung in reinem, wasserfreiem tert.-Butylalkohol vor. Vorzugsweise beträgt die Konzentration des Kaliumsalzes in dem tert.-Butylalkohol 10 bis 15 Gew.-%.

Aus dem Sumpf wird kontinuierlich, vorzugsweise über einen Überlauf, die Kalium-tert.-butoxid-Lösung abgezogen und anschließend auf an sich bekannte Weise, z.B. durch Abdestillieren des Alkohols im Vakuum, das Kaliumsalz isoliert.

Dieses fällt als weißes, feingekörntes, hygroskopisches Pulver hoher Reinheit an.

In einer erfindungsgemäßen Variante wird tert.-Butylalkohol in 9 bis 20-facher Volumenmenge, bezogen auf wässrige KOH, dampfförmig in die Kolonnenmitte bei sonst gleichartiger Verfahrensführung eingespeist. Auf diese Weise kann die Durchsatzrate und damit die Kapazität gesteigert werden.

In einer weiteren erfindungsgemäßen Variante wird bis zur Hälfte des Volumens an tert.-Butylalkohol durch Cyclohexan oder Hexan ersetzt und das Gemisch dieser Lösungsmittel auf den Kolonnenkopf gegeben. Auf diese Weise wird Kalium-tert.-butoxid in der Lösung eines Gemisches aus tert.-Butylalkohol und Cyclohexan (bzw. Hexan) erhalten und in der oben beschriebenen Weise isoliert. Diese Verfahrensweise hat einen positiven Einfluß auf die Beschaffenheit (Körnung) des Produktes und erleichtert die Trocknung. Auch bei dieser Arbeitsweise können die aus dem Sumpf abdestillierten Lösungsmittel der Synthese wieder zugeführt werden.

Beispiel 1

Die Reaktion wird in einer kontinuierlich betriebenen Destillationskolonne, Nennweite 50 mm und 8 m wirksame Füllkörperhöhe durchgeführt. Die Kolonne ist mit V4A-Füllkörpern gefüllt, die sich durch hohe Oberfläche und große Trennwirkung auszeichnen. Der Sumpf der Kolonne ist mit einem Überlauf ausgestattet, an dem die Produktlösung abgezogen wird. Der Kopf der Kolonne ist mit einem Abscheider versehen, der die Abtrennung der wässrigen Phase ermöglicht und die organische Phase auf den Kolonnenkopf zurückführt.

Vor Beginn der Reaktion wird im Sumpf ein Gemisch aus tert.-Butylalkohol und Cyclohexan zum Sieden gebracht, so daß sich in der Kolonne ein Gleichgewicht der beiden Komponenten einstellt. Die Zusammensetzung des Ausgangsgemisches wird so gewählt, daß nach Gleichgewichtseinstellung das Kondensat in der Kolonnenmitte noch ca. 30 Gew.% Cyclohexan enthält. In dieser Vorbereitungsphase wird das im Gemisch spurenweise enthaltene Wasser bereits herausgetrennt. Ist die Kolonne auf diese Weise vorbereitet, wird mit der Reaktion begonnen. Bei kontinuierlicher Zugabe pro Stunde von 18,5 g 50%iger Kalilauge und 117 g tert.-Butylalkohol mit einem Wassergehalt < 0,05% auf den Kopf der Kolonne erhält man eine ca. 15%ige Lösung von Kalium-tert.-butoxid in tert.-Butylalkohol. Die Lösung wird am Rotationsverdampfer bei 40°C und 100 mbar weitgehend eingedampft, die Endtrocknung findet bei 160°C und 10 mbar statt. Das Endprodukt fällt als weißes, feinkörniges Pulver an, dessen KOH-Gehalt 0,4% beträgt.

Beispiel 2

Der Versuch wird analog Beispiel 1 jedoch mit Hexan statt Cyclohexan durchgeführt. Bei stündlicher Zugabe von 17,3 g 50%iger KOH und 103 g tert.-Butylalkohol, Wassergehalt < 0,05%, erhielten wir eine ca. 15%ige Produktionslösung. Das Endprodukt enthält nach Trocknung 0,8% KOH.

Beispiel 3

Der Versuch wird analog Beispiel 1 durchgeführt, jedoch werden 50 Vol.-% der tert.-Butylalkoholmenge durch Cyclohexan ersetzt. Bei stündlicher Zugabe von 10 g 50%iger Kalilauge und 72 g eines Gemisches aus 36 g Cyclohexan und 36 g tert.-butylalkohol erhielten wir eine ca. 11%ige Produktlösung. Das Endprodukt enthält nach Trocknung 0,6% KOH.

## Patentansprüche

1. Verfahren zur kontinuierlichen Herstellung von wasserfreiem Kalium-tert.-butoxid durch Umsetzung von wässriger Kalilauge mit tert.-Butylalkohol in einer mit Füllkörpern gefüllten Destillationskolonne, Abdestillieren des Wassers unter Mitverwendung eines Schleppmittels und Abziehen der im Sumpf der Kolonne anfallenden alkoholischen Lösung des Kalium-tert.-butoxides, wobei aus dieser abgezogenen Lösung anschließend in an sich bekannter Weise das wasserfreie Salz gewonnen wird, dadurch gekennzeichnet, daß

a) als Schleppmittel Cyclohexan oder Hexan eingesetzt wird,

b) der tert.-Butylalkohol in einem solchen Überschuß zu der wäßrigen Kalilauge und dem Schleppmittel eingesetzt wird, daß im Sumpf der Kolonne eine 10 bis 18 Gew.-%ige Lösung von Kalium-tert.-butoxid vorliegt und der Gehalt an tert.-Butylalkohol in dem in der Kolonnenmitte befindlichen Gasgemisch zwischen 50 und 90 Gew.-% liegt und

c) über Kopf bei Temperaturen zwischen 65 und 75 °C ein Gemisch aus Schleppmittel, tert.-Butylalkohol und Wasser abdestilliert wird.

2. Verfahren gemäß Anspruch 1, dadurch gekennzeichnet, daß aus dem über Kopf abdestillierten Gemisch die organische Phase abgetrennt und der Destillationskolonne wieder zugeführt wird.

3. Verfahren gemäß Anspruch 1 oder 2, dadurch gekennzeichnet, daß der tert.-Butylalkohol dampfförmig im oberen Kolonnenteil, vorzugsweise zur Kolonnenmitte hin, zugeführt wird.

4. Verfahren gemäß einem der Ansprüche 1 bis 3, dadurch gekennzeichnet, daß bis zu 50% des tert.-Butylalkohols durch Cyclohexan oder Hexan ersetzt wird.

## Claims

1. Process for the continuous preparation of water free potassium tert.-butoxide by reaction of aqueous potassium hydroxide with tert.-butyl alcohol in a distillation column filled with filler bodies, distillation off of the water with simultaneous use of a carrier agent and withdrawal of the alcoholic solution of the potassium tert.-butoxide collecting in the sump of the column, with the water free salt then being obtained from this withdrawn solution in known manner, characterised in that

a) cyclohexane or hexane is employed as carrier agent,

b) the tert.-butyl alcohol is employed in such an excess to the aqueous potassium hydroxide and the carrier agent that a 10 to 18% by weight aqueous solution of potassium tert.-butoxide is present in the sump of the column and the content of tert.-butyl alcohol in the gas mixture present in the middle of the column lies between 50 an 90% by weight and

c) a mixture of carrier agent, tert.-butyl alcohol and water is distilled off over the head at temperatures between 65 and 75 °C.

2. Process according to claim 1, characterised in that the organic phase is separated off from the mixture distilled off over the head and returned to the distillation column again.

3. Process according to claim 1 or 2, characterised in that the tert.-butyl alcohol is supplied in vapour phase to the upper part of the column, preferably to the middle of the column.

4. Process according to one of claims 1 to 3, characterised in that up to 50% of the tert.-butyl alcohol is replaced by cyclohexane or hexane.

## Revendications

1. Procédé de préparation en continu de tertio-butylate de potassium anhydre, par réaction d'une lessive aqueuse de potasse avec de l'alcool tertio-butylique dans une colonne de distillation emplie de corps de garnissage, départ de l'eau par distillation avec co-utilisation d'un entraîneur et soutirage de la solution alcoolique de tertio-butylate de potassium obtenu en pied de colonne, le sel anhydre étant ensuite obtenu de façon connue en soi à partir de cette solution soutirée, procédé caractérisé en ce que:

a) on utilise comme entraîneur du cyclohexane ou de l'hexane,

b) on utilise l'alcool tertio-butylique en un excès tel par rapport à la lessive aqueuse de potasse et par rapport à l'entraîneur qu'il y ait en pied de colonne une solution de 10 à 18% en poids de tertio-butylate de potassium et que la teneur en alcool tertio-butylique se situe, dans le mélange gazeux se trouvant en mileu de colonne, entre 50 et 90% en poids, et

c) on chasse par distillation, en tête, à des températures comprises entre 65 et 75%, un mélange de l'entraîneur, de l'alcool tertio-butylique et d'eau.

2. Procédé selon la revendication 1, caractérisé en ce qu'on sépare la phase organique du mélange chassé par distillation en tête et en ce qu'on recycle à nouveau cette phase organique vers la colonne de distillation.

3. Procédé selon la revendication 1 ou 2, caractérisé en ce qu'on achemine l'alcool tertio-butylique sous forme de vapeur vers la partie supérieure de la colonne, avantageusement vers le milieu de la colonne.

4. Procédé selon l'une des revendications 1 à 3, caractérisé en ce qu'on remplace jusqu'à 50% de l'alcool tertio-butylique par du cyclohexane ou par de l'hexane.